# EUROPEAN PATENT SPECIFICATION

(11) **EP 0 949 331 B1**
(45) Date of publication and mention of the grant of the patent: **10.01.2007**
(21) Application number: 98905808.6
(22) Date of filing: 05.03.1998
(51) Int. Cl.: C12N 5/06, C12N 5/10, C12N 15/12, G01N 33/48, A61K 35/30, A61K 48/00

(54) **ESTABLISHED MICROGLIA**
Etablierte Mikrogliazellen
Lignée établie de microglie

(30) Priority: 05.03.1997 JP 5044897
(43) Date of publication of application: 13.10.1999
(73) Proprietor: Japan Science and Technology Agency, Kawaguchi-shi Saitama (JP)
(72) Inventor: SAWADA, Makoto, Kasugai-shi Aichi-ken 487-0015 (JP)
(74) Representative: Cresswell, Thomas Anthony
(86) International application number: PCT/JP1998/000949
(87) International publication number: WO 1998/039415

(56) References cited:
- JP-A- 5 049 473
- SUZUMURA AKIO ET AL: "Selective induction of interleukin-6 in mouse microglia by granulocyte-macrophage colony-stimulating factor." BRAIN RESEARCH, vol. 713, no. 1-2, 1996, pages 192-198, XP000982667
- SAWADA MAKOTO ET AL: "Production of interleukin-5 by mouse astrocytes and microglia and culture." NEUROSCIENCE LETTERS, vol. 155, no. 2, 1993, pages 175-178, XP002161941
- MAKOTO SAWADA et al., "Brain Selective Congenic Method with the Use of Isolated Microglia and Established Microglia (in Japanese)", BULLETIN OF THE JAPANESE NEUROCHEMICAL SOCIETY, Vol. 35, No. 3, (1996), pages 704-705, XP002944366.
- L. BITTING et al., "Beta-Amyloid Peptide Secretion by a Microglial Cell Line is Induced by beta-Amyloid-(25-35) and Lipopolysaccharide", JOURNAL OF BIOLOGICAL CHEMISTRY, Vol. 271(27), (1996), p. 16084-16089, XP000999348.
- U. MOENNING et al., "Extracellular Matrix Influences the Biogenesis of Amyloid Precursor Protein in Microglial Cells", JOURNAL OF BIOLOGICAL CHEMISTRY, Vol. 270(13), (1995), pages 7104-7110, XP000999349.
- AKIO SUZUMURA et al., "Functional and Propagational Regulation of Cultured Microglia with the Use of Colony Stimulating Factor (in Japanese)", BULLETIN OF THE JAPANESE NEUROCHEMICAL SOCIETY, Vol. 28, No.1, (1989), pages 52-53, XP002944365.
- D.D. MOSSER et al., "Use of a Dicistronic Expression Cassette Encoding the Green Fuluorescent Protein for the Screening and Selection of Cells Expressing Inducible Gene Products", BIOTECHNIQUES, Vol. 22(1), (Jan. 1997), P. 150-161, XP002151705.
- S. ERIKSSON et al., "Green Fluorescent Protein as a Tool for Screening Recombinant Baculoviruses", JOURNAL OF VIROLOGICAL METHODS, Vol. 59, (1996), p. 127-133, XP001007867.
- S. DELAGRAVE et al., "Red-Shifted Excitation Mutants of the Green Fluorescent Protein", BIO/TECHNOLOGY, Vol. 13, (1995), p. 151-154, XP002033686.

## Description

### Technical Field

The present invention relates to a subcultivatable, established cell line of microglia, a method of separating the same, and use thereof as a pharmaceutical carrier.

Further, we describe a microglia cell line into which an extraneous gene or a drug was introduced, a method of introducing it, and a pharmaceutical composition comprising the same.

### Background Art

There are quite a number of hereditary diseases in the nervous system, which occur due to various causative factors, for example by defect of a single enzyme, etc. or by unknown reasons. Under these circumstances, supplementary therapy is used to cope with a large number of such diseases.

A large number of studies have been made worldwide on the system of selective delivery to the brain. For introducing a gene into the brain in an animal, a method of using neuronphilic viruses as adenovirus vectors is devised, and a system for introducing a gene specifically into neurons is known (Kozarsky, K.F and Wilson, J. M., Curr. Opin. Genet. Dev. 3, 499-503, 1993). A method of using a retrovirus vector is also devised and has succeeded in introducing a gene into hepatic cells, bloodcells, etc. (Mullingan, R. C. , Science 260, 926-932, 1993).

In the brain, however, the blood-brain barrier is present, so it is difficult to conduct supplementary therapy and to introduce an effective drug, and even if a substance (e.g. anticancer drug, DNA etc.) is introduced from a peripheral position, it cannot be introduced specifically into the brain. Therefore, there was no method other than direct injection of the substance by surgical operations.

As a method not involving invasive means such as surgical operations, there is a method of utilizing liposomes, and liposomes rendered capable of introduction into the brain relatively easily by changing their constitutional elements were developed by a Japanese group. However, even in this method too, incorporation of liposomes into the brain is as low as about 1 % based on the injection amount, so this method cannot be said to be specific for the brain.

As described above, the blood-brain barrier is present in the brain, so the brain is almost free of infiltration with cells or a substance from the periphery, thus making it difficult to introduce a drug or a gene into the brain. Actually, infiltration of the normal brain with immunocytes such as T cells and macrophages is hardly observed.

Microglia are cells with macrophage-like properties in the central nervous system, which not only function as immunocompetent cells in inflammatory reaction and viral infection and as phagocytes for removing cells but also play a central role in a cytokine network in the central nervous system (Sawada, M. et al. , Int. J. Dev. Neurosci., 13, 253-264, 1995). Recently, microglia have been revealed to be essential for expression of high-level brain functions such as learning and memorization and considered to be specialized cells having a role specific for the brain. Up to now, it has been considered that microglia originate from monocytes having infiltrated the brain in the perinatal period and are specialized and differentiated.

The microglia can be obtained by primary culture of brain cells. For this primary culture, however, the brain should be excised and purified for use, and primary culture usually requires a period of about 2 weeks, so the procedures are cumbersome. Further, the cells are difficult to proliferate during culture and hard to subculture, so after primary culture, it is extremely difficult to introduce a gene into the microglia to express it therein

In a process of studies on microglia considered as brain-specific macrophage, the present inventors have succeeded in obtaining highly purified microglia and examined the properties, and as a result, we found that unlike the macrophage, microglia have specific affinity for the brain.

Further, the inventors found that microglia are determinatively different from macrophage in respect of affinity for the brain and the ability to infiltrate the brain. Further, the inventors examined the distribution thereof by staining in a method capable of distinguishing the two, and found that microglia are present from an early development stage in the brain. Accordingly, it is considered that microglia are not derived from a monocyte differentiated and matured in the bone marrow, but are derived from a cell group which have a specific affinity for the brain, and infiltrate the brain at an early development stage and come to regulate high-level functions such as cerebral morphogenesis and learning and memorization.

Accordingly, the inventors injected an isolated macrophage and microglia into rat peripheral arteries to compare the two for selective affinity for the brain, and as a result we found that when the microglia labeled with a fluorescent pigment was injected; many fluorescent cells were observed in the brain but hardly observed in the liver. On the other hand, when the macrophage was injected, fluorescent cells were hardly observed in the normal cell, but many fluorescent cells were observed in the liver.

Then, the inventors examined whether a cell strain of microglia established by the inventors can express a gene selectively in the brain by introducing a lacZ expression vector into the cell strain and injecting the resultant cells into a rat blood stream, and as a result, the inventors could detect the activity of β-galactosidase in a section of the rat brain into which the lacZ-expressing cells had been injected. From these results, it was found that the microglia unlike the macrophage is a cell having specific affinity for the brain, and by utilizing this affinity, a specific substance or gene can be introduced via a peripheral blood stream into the brain.

### Disclosure of Invention

That is, the present invention relates to an established cell line of microglia having specific affinity for the brain and phagocytic ability, and having cell growth ability dependent on granulocyte-macrophage colony-stimulating factor (GM-CSF), characterised in that said cell line is subcultivatable in the presence of GM-CSF and has essentially a macrophage-like or globular form in the presence of GM-CSF and a branched form similar to branched microglia present in the brain in the absence of GM-CSF.

Further, the present invention relates to a method of producing the established cell line of microglia which comprises the step of cloning a cell line of microglia from purified microglia through the cell culture in the presence of GM-CSF.

Further, the present invention relates to a pharmaceutical composition comprising the established cell line of microglia.

Further, the present invention relates to the pharmaceutical composition for use in a method of treating cerebral disease.

We also describe a method of separating a subcultivatable established cell line of microglia from microglia cells in the presence of a cytokine, preferably in the presence of colony-stimulating factor (CSF), more preferably in the presence of granulocyte-macrophage colony-stimulating factor (GM-CSF), still more preferably in the presence of IL-3 and/or purified astrocytes.

Also described is a pharmaceutical carrier comprising the established cell line of microglia described above.

We also describe the above-described established cell line of microglia having a gene or a drug introduced into it.

Also described is a pharmaceutical composition comprising the above-described microglia having a gene or a drug introduced into it and a pharmaceutical carrier and in particular to a pharmaceutical composition which is an agent for treatment of cerebral diseases.

We also describe a method for screening or producing a microglia having an extraneous gene introduced into it, comprising introduction of an extraneous gene and a gene expressing a fluorescent protein, preferably a fluorescent protein derived from a jellyfish, into a microglia. As the microglia the conventional microglia can also be used, but the above-described established cell line of microglia is preferably used.

We also describe using the above-described pharmaceutical composition to deliver a drug or gene specifically to the brain.

Hereinafter, the present invention is described in more detail.

### Brief Description of Drawings

Fig. 1 is a photograph showing the form of the established cell line of microglia of the present invention (morphology of the cells).
Fig. 2 is a photograph showing the difference in tissue specificity between the established cell line of microglia of the present invention and macrophages (morphology of the cells).
Fig. 3 shows electrophoresis indicating cytokine expression by stimulation with lipopolysaccharides.
Fig. 4 shows GM-CSF-dependent proliferation of the established cell line of microglia of the present invention.
Fig. 5 is a photograph indicating gene expression in a rat brain (morphology of the cells).
Fig. 6 shows gene expression in a rat brain.
Fig. 7 shows a result of FACS analysis of cells into which a gene was not introduced.
Fig. 8 shows a result of FACS analysis of cells into which GFP was introduced.

### Best Mode for Carrying Out the Invention

The established cell line of microglia of the present invention can be purified from mouse or rat brain cells after primary culture and then separated by the following means from this purified microglia. In addition, because the established cell line of microglia of the present invention is easily handled and has affinity for the brain, a gene or drug can be introduced into the established cell line of microglia and injected into peripheral blood vessels to express the gene in the brain or to deliver the drug to the brain specifically.

Hereinafter, the process for producing the established cell line of microglia of the present invention is described in more detail.

### (1) Purification of Microglia

First, the meninges are removed from collected mouse or rat brains and divided into single cells by use of a pipette, nylon mesh, etc. The mouse and rat are preferably newborn. The mouse includes, but is not limited to, C57BL6, C3H, ICR, Balb/c etc., and the rat includes, but is not limited to, Fisher, Wister, SD, etc.

The resulting cells are plated on a usual animal cell culture medium (e.g. EMEM containing 10 % FCS or CS) and cultured for 10 to 14 days. The medium is exchanged with fresh one every 3 to 4 days.

Then, the cultured cells thus obtained are selected to prepare an established cell line in the subsequent step.

There are microglia called type I and type II, and type I is floating cells which are removed from the culture vessel upon mechanical stimulation of cells after primary culture (that is, by splashing the cells with a medium through a pipette, by shaking the culture vessel, etc.). Type II is cells (adherent cells) not floated by said mechanical stimulation. Since the microglia of the present invention belongs to type II, purified microglia can be selected in the following manner.

The adherent cells which do not float by the above mechanical stimulation are treated with trypsin-EDTA, then divided into single cells, plated on a non-treated plastic dish (non-coat plastic vessel), and allowed to adhere to it. Generally, a conventional culture vessel is treated with chemicals so as to be positively charged, but a dish not subjected to this treatment should be used in order to obtain the adherent cells. After incubation in a CO₂ incubator at 37 °C for 1 hour, cells floating in the medium after mechanical stimulation are removed, and cells capable of proliferation on the vessel are recovered by Rubber Policeman, etc., and the same procedure is repeated twice to obtain cells to be used in establishing a cell line of microglia. Although the purified microglia thus obtained is of adequate purity, cell sorter, etc. can also be used to further improve purity.

### (2) Separation of Established Cell Line of Microglia

To separate the subcultivatable established cell line of microglia of the present invention from the purified microglia obtained in the manner described in (1) above, the purified microglia is cloned through culture in the presence of a cytokine, preferably colony-stimulating factor (CSF), more preferably granulocyte-macrophage colony-stimulating factor (GM-CSF). As the cytokine allowed to be present during culture, GM-CSF may be used singly, but IL-3 and/or a supernatant derived from purified astrocytes may be allowed to be present in addition to GM-CSF. The cytokine used may be the naturally occurring or genetic recombinant one. Specific procedures are for example as follows:

The purified microglia obtained in (1) above is plated on a vessel of about 10 cm diameter and cultured for 7 to 10 days in the presence of genetic recombinant granulocyte-macrophage colony-stimulating factor (rGM-CSF). After culture, the cells are recovered, and using limiting dilution, they are further cultured for 4 to 10 weeks in the presence of rGM-CSF. Then, cells having formed a single colony in each well in the test plate are released by Rubber Policeman whereby the cloned cells are sorted and separated to finally give an established cell line of microglia.

The established cell line of microglia thus obtained has the following properties.

### (a) Form

When observed after staining with a fluorescent pigment under a fluorescence microscope or without staining under a phase contract microscope, the cells have a macrophage-like or globular form in the presence of rGM-CSF, or in the absence of rGM-CSF, a branched form similar to branched microglia present in the brain. Otherwise the cells have both of the above forms.

### (b) Functional Characteristics

Upon administration into an artery of a mouse, the established microglia of the present invention moves specifically to the brain, indicating that it has specific affinity for the brain. Further, when stimulation is given with lipopolysaccharides the microglia produces interleukin- 1 (IL-1) and interleukin-6 (IL-6). Upon stimulation with interferon γ, it produces IL-5. This property is different from that of macrophages because macrophages do not express IL-5 upon stimulation with IFN-γ. When the phagocytic ability of the cell line of the present invention is examined using incorporation of a fluorescent pigment as an indicator, it has a strong phagocytic ability. The established cell line of microglia of the present invention has a phagocytic ability which is hundreds to thousands times higher than that of astrocytes.

### (c) Cell Growth Ability

Because the established microglia of the present invention is not proliferated after removal of rGM-CSF from the medium, it is proliferated depending on GM-CSF.

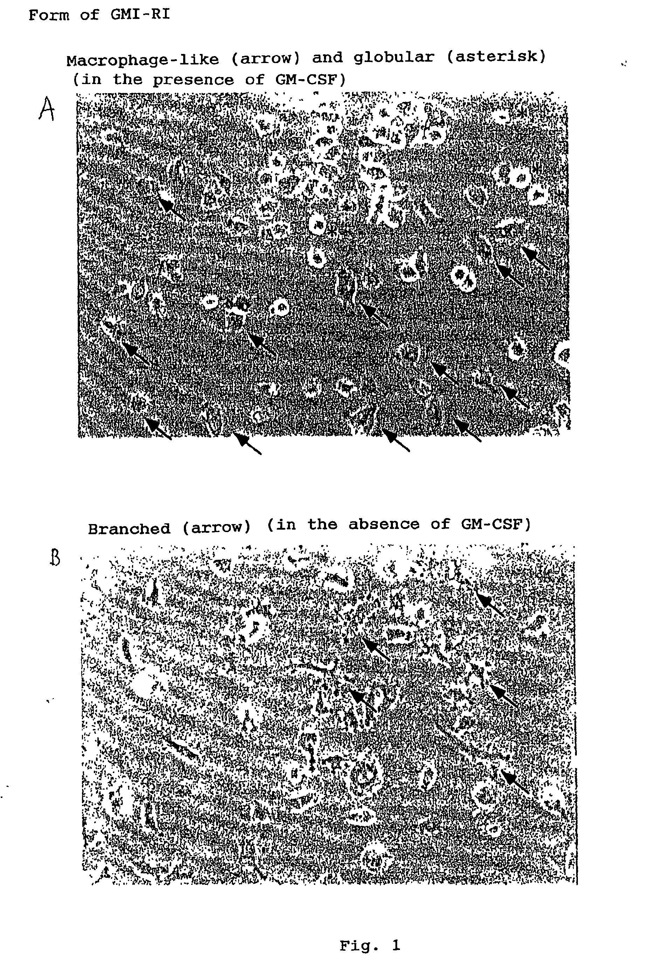

The form and characteristics of the established microglia of the present invention are exemplified in specific examples in Figs. 1 to 4.

### (3) Introduction of Gene into the Established Cell Line of Microglia

Introduction of a gene into the established cell line of microglia of the present invention is important for expressing the gene specifically in the brain. A desired gene can be obtained by known cloning means, and any commercially available gene can also be used, and the type of gene is particularly not limited.

As a means of efficiently introducing a gene into the established cell line of microglia, there is for example a method of using DOTAP (Boehringer-Mannheim Co.). That is, there is a method of culturing the established cell line of microglia together with a desired gene in a gene-introducing medium containing DOTAP. They are cultured in a CO₂ incubator at 37 °C for 16 to 24 hours and further cultured together with rGM-CSF for 30 to 72 hours (preferably for 48 hours) in a medium for culturing microglia.

In addition to the above-described means, the method of introducing the gene into the microglia includes conventional means such as calcium phosphate method, DEAE dextran method, lipofection method, electroporation method, particle gum method, etc

Out of known methods of separating a strain stably expressing an introduced gene, the most generally used method is a method wherein a drug resistance gene is introduced simultaneously with a desired gene into cells and expressed therein, and cells other than those cells coming to express the gene stably and constantly by incorporating the gene into their chromosomal DNA are perished by chemical treatment and excluded from the culture, whereby the desired cells are separated.

When this screening method is applied to the microglia, the microglia coming to express the introduced gene stably and constantly recognize dead cells thus expressing a strong phagocytic ability and simultaneously undergoing activation to lose their cell growth ability. Accordingly, the method of introducing a gene in the present invention is preferably a method wherein an expression vector modified so as to be capable of expressing a fluorescent protein, preferably green florescent protein (GFP) derived from an aurelia, in higher animals is used in place of a drug resistance gene, and by using a difference in fluorescence intensity, the microglia is separated as the desired cells exhibiting stable and constant expression.

Whether the cells having the gene introduced into them have reached the brain and whether the gene has been expressed can be confirmed in the following manner: The cells to be introduced are stained with a fluorescent pigment specific for phagocytes. After introduction of the cells into an animal, the brain is excised and frozen, from which a section of about 8 microns in thickness is prepared and examined for fluorescent cells under a fluorescence microscope, or the section is activity-stained with a substrate for the introduced gene.

The cells can also be confirmed using magnetic resonance image (MRI), positron emission tomography (PET) etc. For example, contrast media, etc. for MRI may be incorporated into the cells which are then injected into an animal so that the cells can be monitored in the animal. According to these methods, it is not necessary to kill the animal, and the cells can be monitored easily in a non-invasive manner.

### Examples

Hereinafter, the present invention is described in more detail by reference to Examples.

### Example 1. Separation of Established Clone of Microglia

### (1) Isolation of Microglia

Brains were excised from newborn mice (C57BL6, op/op) and newborn rats (Fisher), and meninges were removed in an ice-cold microglia culture medium (referred to as Mi medium; Eagle's MEM containing 10 % bovine serum, 0.2 % glucose and 5 µg/ml bovine insulin). The cells of meninges were divided into single cells with a Pasteur pipette or nylon mesh and then cultured in Mi medium. For the cells from the mouse brains, 20 ml Mi medium was used per one brain, and for the cells from the rat brains, 40 ml Mi medium was used per one brain, and the former cells were incubated in 2 culture vessels of 10 cm diameter and the latter cells in 4 culture vessels of 10 cm diameter in a CO₂ incubator (5 % CO₂, 95 % air) at 37 °C for 10 to 14 days. The medium was exchanged with fresh one every 3 to 4 days

When phase-bright round cells (PBRCs) appeared, the PBRCs were removed by mechanical shaking, and the remaining cells were removed with 200 U/ml trypsin-0.02 % EDTA and incubated in a non-coat plastic vessel at 37 °C for 30 minutes. The cells which adhered to the non-coat plastic vessel were washed twice with Mi medium, and the cells were then removed and recovered. The same procedure was repeated further twice to give purified microglia.

### (2) Separation of Clones

1×10⁵ purified microglia cells obtained in (1) above were plated on a 10 cm vessel and cultured for 7 days in Mi medium in the presence of rGM-CSF (GenzymeCo.).

The cells were recovered and counted, and clones were obtained from the cells in the following manner using limiting dilution. The cells were put to each well on a 96-well plate (Falcon Co.) at a density of 0.5 cell/well (in 100 µl) and cultured for about 3 weeks in the presence of 2 ng/ml mouse gene recombinant GM-CSF (Genzyme Co.). Each well was examined for the presence of the clone, and the target clones were separated.

As a result, five kinds of established microglia (Ra2, GMI-M6-1, GMI-M6-3, GMI-M5-2, GMI-MF11) derived from the mouse brains and one kind (GMI-Rl) from the rat brains were obtained.

Among these, the established cell lines of microglia, Ra2 and GMI-R1, have been designated as "mouse microglia Ra2" and "rat microglia GMI-R1" and deposited as FERM P-16109 and FERM P-16110 respectively with the National Institute of Bioscience and Human-Technology, Agency of Industrial Science and Technology, Japan.

The properties of the resulting clones were then examined.

### (a) Form

When GMI-RI incubated in the presence or absence of rGM-CSF was observed under a phase contrast microscope, it had a macrophage-like or globular form in the presence of rGM-CSF (Fig. 1 A) and a branched form in the absence of rGM-CSF (Fig. 1 B).

### (b-1) Functional Characteristics (affinity for the brain)

The established cell line of microglia GMI-R1 of the present invention was allowed to adhere to a plastic vessel. Separately, a fluorescent pigment PKH26 (Zynaxis Co.) prepared in a phagocyte-staining solution (Diluent B, Zynaxis Co.) and 10 % serum were mixed at a ratio of 1 : 1 and added to the above vessel, and GMI-RI was stained with the fluorescent pigment at 37 °C for 15 minutes (Ishihara. S., Sawada, M. et al., Exp. Neurol., 124, 219-230, 1993).

The cells were recovered and 2×10⁶ cells were injected into an artery in the armpit of each of 5-week-old rats from the same strain (Fisher). 48 hours and 1, 2, and 3 weeks after injection, each organ was excised from the rats and frozen in a solution of OCT (Tissue Tek Co.).

Microglia GMI-R1, and macrophages which was isolated for comparison from the abdomen of a rat of the same strain (Fisher) by washing it with cold PBS, were labeled respectively with a fluorescent pigment specific for phagocytes and then injected into armpit arteries of rats, and tissue sections were prepared for examination of the tissue orientation.

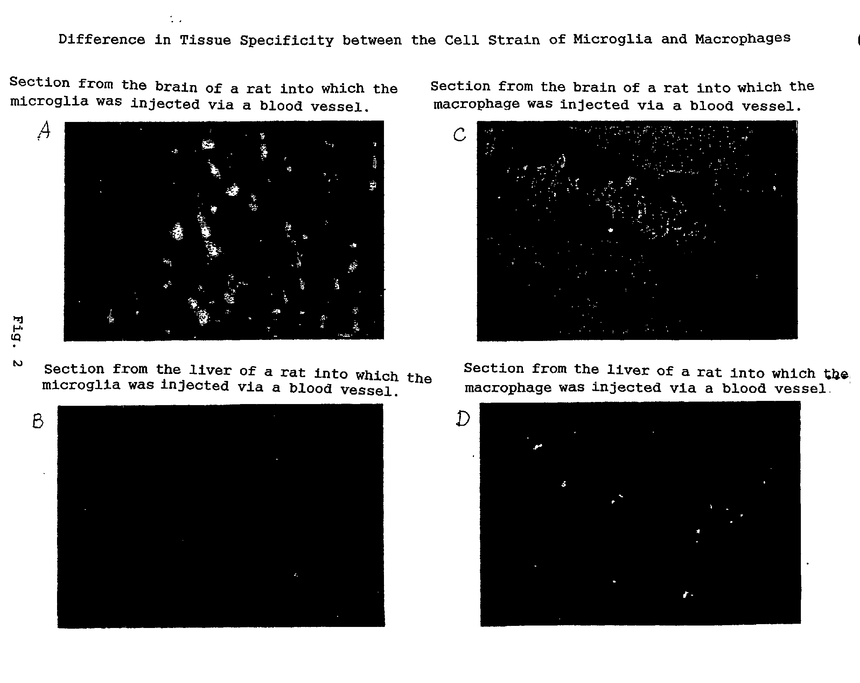

After the established cell line of microglia of the present invention was injected, many fluorescent cells were observed in normal brain cells (Fig. 2 A) but not observed in the liver (Fig. 2 B). On the other hand, after the macrophages were injected, fluorescent cells were hardly observed in normal brain cells (Fig. 2 C), while many fluorescent cells were observed in the liver (Fig. 2 D).

Accordingly, the established cell line of microglia of the present invention possessed specific affinity for the brain. (b-2) Functional Characteristics (ability to produce IL-1 and IL-6)

1×10⁶ Ra2 cells were plated onto a 6 cm culture vessel, and total RNA was extracted from the cells stimulated with lipopolysaccharides for 12 hours and from the cells not stimulated by using RNeasy (Qiagen Co.), and 2 µg of the RNA was used to prepare a cDNA mixture by reverse transcriptase (BRL). PCR was carried out using the resulting cDNA as a template, where an IL-1 specific synthetic primer and IL-6 specific synthetic primer having the following sequences were used. IL-1 specific synthetic primer (Sawada et al., Int. J. Dev. Neurosci, 13, 253-264, 1995):
Sense chain: 5'-ATGGCAACTGTTCCTGAACTCAACT-3' (SEQ ID NO: 1)
Antisense chain: 5'-CAGGACAGGTATAGATTCTTTCCTTT-3' (SEQ ID NO: 2)
IL-6 specific synthetic primer (Sawada et al., Brain Res. 583, 296-299, 1992):
Sense chain: 5'-ATGAAGTTCCTCTCTGCAAGAGACT-3' (SEQ ID NO: 3)
Antisense chain: 5'-CACTAGGTTTGCCGAGTAGATCTC-3' (SEQ ID NO: 4)

In PCR, 30 cycles each consisting of reaction at 55 °C for 1 minute, 72 °C for 2 minutes and 94 °C for 1 minute were carried out (Omnigene from HYBRID Co., Ltd. was used). After PCR, the amplification product was subjected to agarose gel electrophoresis to examine gene expression.

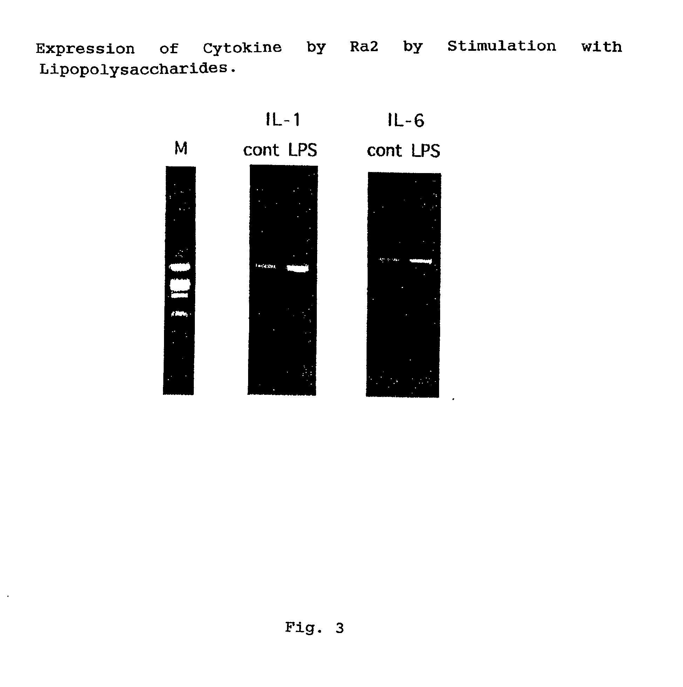

As a result, it was found that Ra2 increased expression of IL-1 and IL-6 (Fig. 3, "LPS" lanes). In Fig. 3, "M" is a molecular weight marker and "cont" is control (not stimulated).

Production of IL-1 and IL-6 was also confirmed by ELISA. Further, a culture supernatant of the established microglia of the present invention after stimulation with lipopolysaccharides was added to MH60 cells proliferating depending on IL-6 or to D10 cells proliferating depending on IL-1, followed by incubation, and whether the MH60 cells and D10 cells were proliferated or not was examined.

As a result, it was found that both the cells proliferated in the presence of the culture supernatant of the established microglia of the present invention stimulated with the lipopolysaccharides.

### (c) Cell Growth Ability

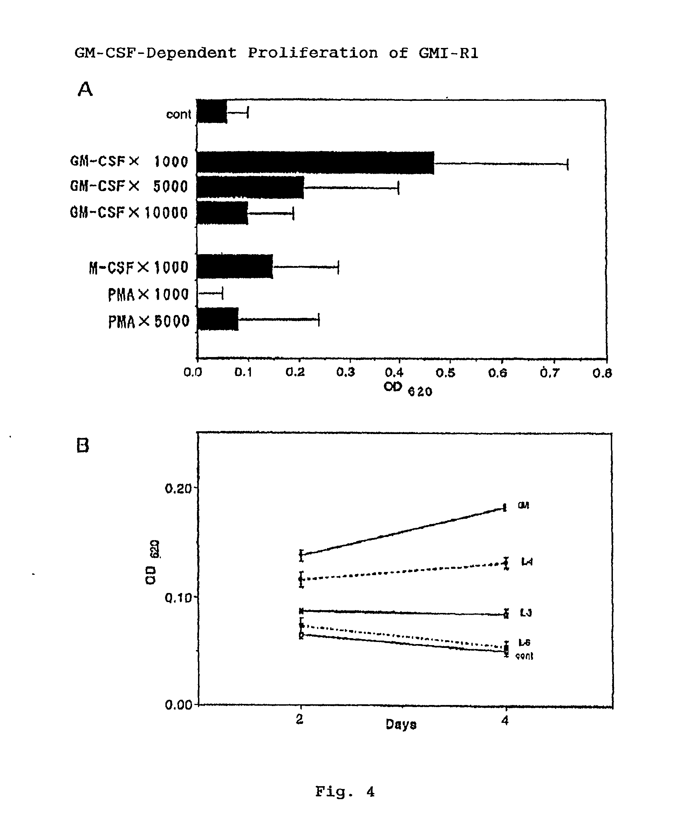

5×10⁴ GMI-R1 cells were plated on a 96-well test plate, and 2 µg/ml rGM-CSF was added to it so as to be diluted 1000-, 5000- and 10000-fold respectively, and the cells were incubated for 4 days and then subjected to MTT assays. As a control, 400 µ/ml human M-CSF (The Green Cross Corporation) was diluted 1000-fold and 0.1 mg/ml PMA (phorbol myristate acetate) was diluted 1000- or 5000-fold, and these were used as the control (Fig. 4 A).

Separately, rGM-CSF diluted 5000-fold, and 100 µ/ml each of mouse IL-3, IL-4 and IL-6 (any of which were produced by Genzyme Co.), were examined in a comparative test. Two days and four days after addition of the respective reagents, MTT assays were carried out (Fig. 4 B).

As a result, it was found that GMI-R1 was proliferated depending on rGM-CSF.

### Example 2. Introduction of a Gene into the Microglia of the present invention

Vector ptkβ (Clonetech Co.) for expression of lac Z gene derived from E . coli and DOTAP lipid (Boehringer-MannheimCo.) were mixed to be a final concentration of 1 µg/ml . The mixture was mixed with a serum-containing medium, then added to the established cell line of microglia of the present invention and treated for 16 hours. As the control, the established microglia of the invention into which the gene was not introduced, and macrophages obtained in the same manner as in Example 1, were used.

Then, the cells were further cultured for 48 hours in a usual medium (EMEM plus 10% FCS) and then stained with the fluorescent pigment as described in Example 1 (b-1) in order to examine whether the gene was delivered to and expressed in the brain, as follows: An artery in the left armpit of a mature rat (250 to 300 g) under anesthesia with Nembutal was exposed. After hemostatic treatment, a cannula was inserted into the artery and used to inject 1 to 2×10⁶ cells into the rat. After injection, the incision site was sutured and the rat was allowed to recover.

48 hours after the cells were injected, the brain was excised from the rat and 3 successive frozen sections of the brain were prepared and observed respectively under a fluorescence microscope. Further, staining and quantification of β-galactosidase activity was carried out in the following manner.

One of the three sections was fixed in 0.5 % glutaraldehyde and subjected to activity staining with Xgal as substrate according to the method of Lim et al. (Bio Techniques 7, 576-579, 1989). To quantify the activity, one of the sections was homogenized in a dissolving buffer by ultrasonication and examined for its activity with a commercial kit (Galacto Light; Boehringer-Mannheim).

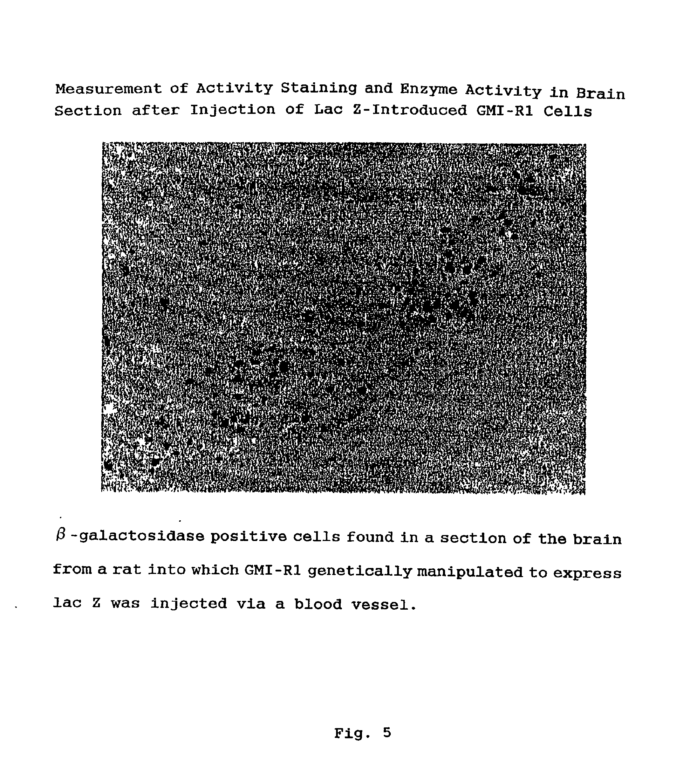

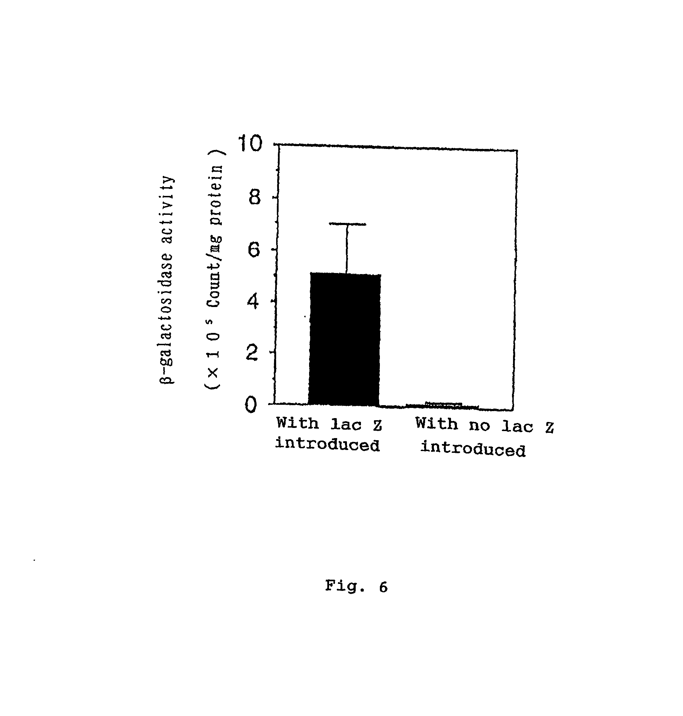

As a result, it could be confirmed that in case the gene was introduced into the established microglia of the present invention, lacZ-positive cells were present in the section from the rat brain (Fig. 5). Further, as a result of quantification of β-galactosidase activity by the chemiluminescence method, considerably higher activity was detected in the rat brain section into which the E. coli-derived gene lac Z expression vector was introduced than the counterpart into which the gene was not introduced (Fig. 6).

### Example 3. Introduction of Chemical Substance into the Microglia of the invention, and Specific Introduction Thereof into the Brain

The fluorescent pigment PKH26 used in Example 1 forms granules in diluent B. The microglia cell strain of the present invention incorporates these granules specifically and transfers them to the brain, so the fluorescent pigment PKH26 was used as a model of chemical substance (anti-tumor drug).

As a result, when the established cell line of microglia of the present invention was introduced, many fluorescent cells were observed in normal brain cells but not observed in the liver. It therefore follows that the microglia of the present invention transfers the chemical substance (drug) into the brain specifically.

### Example 4. Introduction of Gene with GFP

1×10⁶ GMI-R1 cells were plated on a Petri dish, and 16 hours later, 10 µg GFP expression vector pEGFP (Clonteck Co.) was added to a gene-introducing medium, with which the previous medium was then exchanged, and the cells were cultured at 37 °C for 24 hours in a CO₂ incubator and then cultured together with rGM-CSF for additional 7 days in a medium for microglia.

After culture for 7 days, the cells were suspended, and those cells exhibiting fluorescence intensity which was at least 100 times as high as that of the cells into which the gene had not been introduced were fractionated and concentrated by a fluorescent activated cell sorter (FACS) , FACS Calibur produced by Becton-Dickinson and then cultured together with rGM-CSF in a medium for microglia.

The same procedure was repeated additionally twice every 7 days, whereby almost 90 % or more cells could be recovered in a fraction having about 100-fold fluorescence, and these cells were put to a TP96 test plate at a density of 1 cell/well by limiting dilution of the cells and further cultured together with rGM-CSF in a medium for microglia. The microglia coming to expressing the introduced gene pEGFP stably and constantly could thereby be separated.

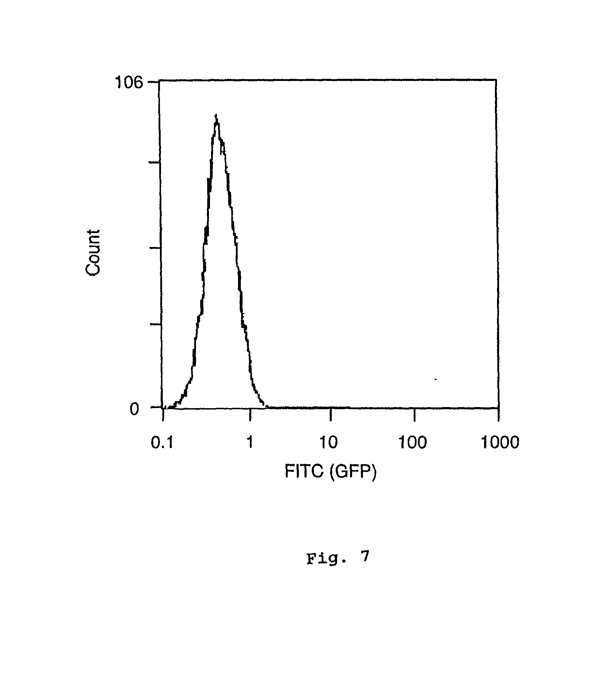

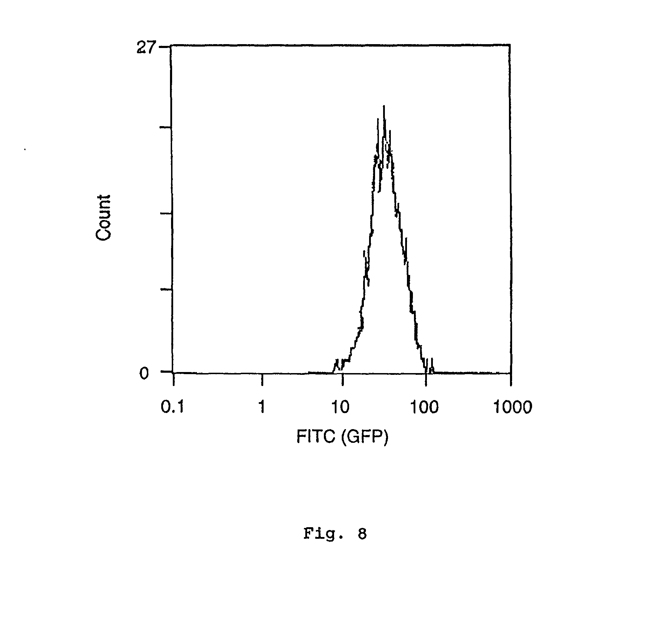

One example of results in FACS analysis as to how the cells could be separated by this method is shown in the drawings. Fig. 7 shows a result of FACS analysis of the cells into which the gene was not introduced, while Fig. 8 shows a result of FACS analysis of the cells into which GFP was introduced.

### Industrial Applicability

According to the present invention, there is provided an established cell line of microglia having specific affinity for the brain. The established cell line of microglia according to the present invention is useful not only as a carrier for introducing a gene into the brain, but also as a carrier four introducing a chemical substance such as drug specifically into the brain.

### SEQUENCE LISTING

SEQ ID NO: 1
   LENGTH: 25 base pairs
   TYPE: nucleic acid
   STRANDEDNESS: single
   TOPOLOGY: linear
   MOLECULAR TYPE: other nucleic acid (synthetic-DNA)
   SEQUENCE DESCRIPTION: SEQ ID NO.I:
   ATGGCAACTG TTCCTGAACT CAACT
SEQ ID NO: 2
   LENGTH: 26 base pairs
   TYPE: nucleic acid
   STRANDEDNESS: single
   TOPOLOGY: linear
   MOLECULAR TYPE: other nucleic acid (synthetic DNA)
   SEQUENCE DESCRIPTION: SEQ ID NO 2:
   CAGGACAGGT ATAGATTCTT TCCTTT
SEQ ID NO: 3
   LENGTH: 25 base pairs
   TYPE: nucleic acid
   STRANDEDNESS: single
   TOPOLOGY: linear
   MOLECULAR TYPE: other nucleic acid (synthetic DNA)
   SEQUENCE DESCRIPTION: SEQ ID NO 3:
   ATGAAGTTCC TCTCTGCAAG AGACT
SEQ ID NO: 4
   LENGTH: 24 base pairs
   TYPE: nucleic acid
   STRANDEDNESS: single
   TOPOLOGY: linear
   MOLECULAR TYPE: other nucleic acid (synthetic DNA)
   SEQUENCE DESCRIPTION: SEQ ID NO 4:
   CACTAGGTTT GCCGAGTAGA TCTC.

## Claims

1. An established cell line of microglia having specific affinity for the brain and phagocytic ability, and having cell growth ability dependent on granulocyte-macrophage colony stimulating factor (GM-CSF), **characterised in that** said cell line is subcultivatable in the presence of GM-CSF and has essentially macrophage-like or globular form in the presence of GM-CSF and a branched form similar to branched microglia in the brain in the absence of GM-CSF.

2. A method of producing the established cell line of microglia of claim 1, which comprises the step of cloning a cell line of microglia from purified microglia through the cell culture in the presence of GM-CSF.

3. The method according to claim 2, wherein the GM-CSF is a genetic recombinant one.

4. The method according to claim 2 or 3, wherein the cell culture is carried out in the presence of IL-3 and/or a culture supernatant of purified astrocytes in addition to the GM-CSF.

5. A pharmaceutical composition comprising an established cell line of microglia as defined in claim 1.

6. The pharmaceutical composition according to claim 5 for use in a method of treating cerebral disease.

## Patentansprüche

1. Etablierte Zelllinie der Mikroglia mit einer spezifischen Affinität für die Gehirn- und Phagozytentätigkeit, und mit Zellwachstumsfähigkeit in Abhängigkeit vom Granulozyten-Makrophagen-Kolonie-stimulierenden Faktor (GM-CSF), **dadurch gekennzeichnet, dass** die Zelllinie in Gegenwart von GM-CSF subkultivierbar ist und im wesentlichen eine Makrophagen-artige oder globuläre Form in Gegenwart von GM-CSF und eine verzweigte Form ähnlich der verzweigten Mikroglia im Gehirn in Abwesenheit von GM-CSF aufweist.

2. Verfahren zum Produzieren einer etablierten Zelllinie der Mikroglia nach Anspruch 1, welches den Schritt des Klonierens einer Zelllinie der Mikroglia aus gereinigter Mikroglia durch die Zellkultur in Gegenwart von GM-CSF umfasst.

3. Verfahren nach Anspruch 2, wobei der GM-CSF ein genetisch rekombinierter ist.

4. Verfahren nach Anspruch 2 oder 3, wobei die Zellkultur in Gegenwart von IL-3 und/oder einem Kulturüberstand von gereinigten Astrozyten, zusätzlich zum GM-CSF, durchgeführt wird.

5. Pharmazeutische Zusammensetzung, umfassend eine etablierte Zelllinie der Mikroglia, wie in Anspruch 1 definiert.

6. Pharmazeutische Zusammensetzung nach Anspruch 5 für die Anwendung bei einer Methode zur Behandlung einer zerebralen Erkrankung.

## Revendications

1. Lignée cellulaire établie de microglie dotée d'affinité spécifique pour le cerveau et de capacité phagocytaire, et dotée d'une capacité de croissance cellulaire dépendant du facteur de stimulation des colonies de granulocytes-macrophages (GM-CSF), **caractérisée en ce que** ladite lignée cellulaire est susceptible de subculture en présence de GM-CSF et possède une forme essentiellement globulaire ou analogue aux macrophages en présence de GM-CSF et une forme ramifiée similaire à la microglie ramifiée dans le cerveau en l'absence de GM-CSF.

2. Procédé de production de la lignée cellulaire établie de microglie de la revendication 1, qui comprend l'étape consistant à cloner une lignée cellulaire de microglie à partir de microglie purifiée par le biais de la culture des cellules en présence de GM-CSF.

3. Procédé selon la revendication 2, dans lequel le GM-CSF est un recombinant génétique.

4. Procédé selon la revendication 2 ou 3, dans lequel la culture cellulaire s'effectue en présence d'IL-3 et/ou d'un surnageant de culture d'astrocytes purifiés en sus du GM-CSF.

5. Composition pharmaceutique comprenant une lignée cellulaire établie de microglie telle que définie à la revendication 1.

6. Composition pharmaceutique selon la revendication 5 pour une utilisation dans un procédé de traitement de maladies cérébrales.
